# FASCICULE DE BREVET EUROPEEN

(11) **EP 0 773 012 B1**
(45) Date de publication et mention de la délivrance du brevet: **26.11.1997**
(21) Numéro de dépôt: 96402247.9
(22) Date de dépôt: 22.10.1996
(51) Int. Cl.: A61H 9/00, A61K 7/48, B65B 31/00

(54) **Dispositif de brumisation d'eau minéralisée**
Vorrichtung zum Vernebeln von Mineralwasser
Means for atomizing mineral water

(30) Priorité: 30.10.1995 FR 9512788
(43) Date de publication de la demande: 14.05.1997
(73) Titulaire: L'OREAL, 75008 Paris (FR)
(72) Inventeur: Blondeel, Gilles, 93600 Aulnay-sous-Bois (FR); Le Calvez, Nicole, 91140 Villebon sur Yvette (FR)
(74) Mandataire: Lhoste, Catherine

(56) Documents cités:
- BR-A- 7 808 500

## Description

L'invention a pour objet un nouveau dispositif de brumisation d'eau minéralisée et plus spécialement d'eau minérale ou thermale, exempt de système mécanique de propulsion.

Le conditionnement des eaux minérales et thermales en récipients aérosols individuels portables, répond à une demande grandissante du public, de pouvoir bénéficier des propriétés physiologiques, voire thérapeutiques, de ces eaux. En effet, la vaporisation sur la peau d'eaux minérales ou thermales, est un moyen qui permet d'assurer une dispersion dans l'air, fine et régulière, sous la forme d'un brouillard, de particules d'eau, et fournit à la peau, outre une agréable sensation de fraicheur, un apport en sels minéraux et en oligo-éléments. Ainsi, grâce à un appareillage très simple, facile à manipuler et à transporter, l'utilisateur peut bénéficier d'un traitement particulièrement bénéfique à la peau et au bien-être de l'utilisateur.

Il est bien connu de l'homme du métier d'utiliser un gaz, comme l'azote, pour pressuriser un dispositif de brumisation , comme décrit par exemple dans : 〈〈 Le conditionnement des eaux sulfurées en emballages aérosols permettant leur utilisation individuelle 〉〉, F.Clanet, Presse thermale et climatique, 1986, 123, n°1.

On connaît également, en particulier par le document BR-7808500, des dispositifs de brumisation d'eau minéralisée dont le gaz propulseur est de l'azote. De tels dispositifs permettent une brumisation fine de l'eau et une bonne tolérance du brumisat par la peau.

Toutefois, certaines eaux fortement minéralisées, en particulier les eaux ayant une forte concentration en carbonates ou en bicarbonates, donnent des précipités de ces ions, lorsqu'elles sont placées sous pression d'azote. Cette précipitation de sels minéraux a pour conséquence une modification de la composition chimique de l'eau à brumiser et donc une altération de ses propriétés et de son action sur la peau. Cette précipitation entraîne également un risque de bouchage du dispositif de brumisation.

Le brevet spécial de médicament n°3574 décrit l'utilisation de gaz carbonique dans des récipients aérosols munis de systèmes mécaniques de propulsion. Toutefois, l'utilisation de ce gaz dans un dispositif individuel de brumisation, exempt de système mécanique de propulsion, ne peut pas satisfaire les exigences du consommateur. En effet, en fin de brumisation, le dioxyde de carbone provoque la formation de gouttelettes ou d'un jet liquide, à la place du brumisat attendu ; des fuites liquides au niveau du diffuseur, tout comme la projection d'un jet désagréable pour le manipulateur, sont des inconvénients qui ne permettent pas de commercialiser un tel dispositif.

On définit le brumisat comme un nuage de particules, ayant une taille comprise entre 50 µm et 120 µm.

Enfin, selon l'art antérieur, une étape importante et coûteuse de la fabrication d'un dispositif de brumisation d'eau minérale ou thermale, réside dans la décontamination de ce dispositif après son remplissage en gaz et en eau. Cette décontamination est faite habituellement par des méthodes physiques telles que les traitements thermiques ou ionisants.

Aussi, c'est avec étonnement que la demanderesse a constaté que l'utilisation d'un dispositif aérosol individuel, pressurisé par un mélange gazeux contenant de l'azote et du dioxyde de carbone, permettait de remédier à l'ensemble de ces inconvénients, tout en assurant une meilleure tolérance de l'eau brumisée par la peau. En effet, l'utilisation d'un dispositif aérosol selon l'invention, pressurisé par un mélange gazeux contenant de l'azote et du dioxyde de carbone n'entraîne pas la formation de précipités minéraux, en particulier de carbonates ou de bicarbonates dissous dans l'eau, ni la formation de jets en fin de brumisation. De plus, ce dispositif permet de réduire et de maintenir à un niveau très faible la teneur en bactéries de l'eau sans avoir recours à un procédé physique de décontamination, et ce, de façon durable dans le temps. Enfin, ce mélange gazeux agit sur le pH de l'eau en le rapprochant de celui de la peau.

De façon plus précise, l'invention se rapporte à un dispositif de brumisation d'eau minéralisée, constitué d'un récipient contenant l'eau minéralisée et un gaz propulseur, d'une valve et d'un moyen de diffusion, caractérisé en ce que le gaz propulseur est un mélange essentiellement constitué d'azote et de dioxyde de carbone.

Le dispositif objet de la présente invention s'applique aux eaux minéralisées en général, et tout particulièrement aux eaux minérales ou thermales. En général, une eau minérale est propre à la consommation, ce qui n'est pas toujours le cas d'une eau thermale. Chacune de ces eaux contient, entre autre, des minéraux solubilisés et des oligo-éléments. Ces eaux sont connues pour être employées à des fins de traitement spécifique selon les oligo-éléments et les minéraux particuliers qu'elles contiennent, tels que l'hydratation et la désensibilisation de la peau ou le traitement de certaines dermatoses. Par eaux minérales ou thermales, on désignera non seulement les eaux minérales ou thermales naturelles, mais également des eaux minérales ou thermales naturelles enrichies en constituants minéraux et/ou en oligo-éléments supplémentaires, ainsi que des solutions aqueuses minérales et/ou oligo-élémentaires préparées à partir d'eau purifiée (déminéralisée ou distillée).

Une eau thermale ou minérale naturelle utilisée selon l'invention peut, par exemple être choisie parmi l'eau de Vittel, les eaux du bassin de Vichy, l'eau d'Uriage, l'eau de la Roche Posay, l'eau de la Bourboule, l'eau d'Enghien-les-Bains, l'eau de Saint Gervais-les-Bains, l'eau de Néris-les-Bains, l'eau d'Allevar-les-Bains, l'eau de Digne, l'eau de Maizières, l'eau de Neyrac-les-Bains, l'eau de Lons-le-Saunier, les Eaux Bonnes, l'eau de Rochefort, l'eau de Saint Christau, l'eau des Fumades et l'eau de Tercis-les-bains, l'eau d'Avene.

Les eaux minéralisées auxquelles s'applique l'invention peuvent, en outre, contenir des adjuvants habituels dans les domaines dermatologique et/ou cosmétique, tels que les conservateurs, les antioxydants, les parfums, les filtres, les matières colorantes, les actifs hydrophiles ou lipophiles. Ces adjuvants doivent être choisis parmi ceux susceptibles d'être distribués sous la forme d'un spray et ne doivent pas entraîner le bouchage du dispositif, en particulier du diffuseur.

Les actifs pour la peau peuvent être des actifs anti-âge, des actifs anti-rides, des hydratants ou des humectants, des actifs dépigmentants, des actifs anti-radicaux libres (espèces radicalaires de l'oxygène), des actifs nutritifs, des actifs protecteurs, des actifs restructurants, des actifs raffermissants, des actifs antiacnéiques, des actifs exfoliants, des actifs émollients ou encore des actifs traitants des maladies de peau comme les mycoses, les dermites, le psoriasis, etc. Ces actifs sont utilisés, selon leur nature, dans les proportions habituelles et par exemple de 0,01 % à 10 % en poids par rapport au poids total de la composition.

Comme actifs anti-acnéiques, anti-âge, anti-ride, hydratants, exfoliants, on peut citer plus particulièrement les α-hydroxy-acides (acides glycolique, lactique, malique, citrique...).

Comme anti-oxydants on peut citer par exemple les sulfites.

Selon l'invention le gaz propulseur employé dans le dispositif de brumisation est un mélange essentiellement constitué d'azote (N₂) et de dioxyde de carbone (CO₂) et, de façon préférentielle, ce gaz propulseur est constitué essentiellement de N₂ et de CO₂.

Des essais de brumisation ont été réalisés avec des dispositifs chargés de mélanges gazeux comprenant des pourcentages en volume d'azote et de dioxyde de carbone variables. Ces essais ont permis de montrer qu'avec plus de 30% d'azote, compté en volume par rapport au volume gazeux total, on évitait la formation d'un jet en fin de brumisation du dispositif.

D'autre part il a été constaté qu'avec au moins 40% de dioxyde de carbone, compté en volume par rapport au volume gazeux total, on évitait la formation d'un précipité de sels minéraux.

De façon préférentielle, le gaz employé dans le dispositif objet de l'invention est un mélange d'azote et de dioxyde de carbone dont les pourcentages en volume par rapport au volume gazeux total, satisfont la relation:

40/60 < %N₂/%CO₂ < 60/40 et %N₂ + %CO₂= 100 (Rel 1)

De façon encore plus préférentielle on choisit:

%N₂ = %CO₂= 50 (Rel II)

L'eau minérale ou thermale à laquelle s'applique l'invention peut avoir une concentration en carbonates ou en bicarbonates élevée, comme par exemple, l'eau de Vittel, les eaux du bassin de Vichy, l'eau d'Uriage, l'eau de la Roche Posay, l'eau de la Bourboule, l'eau d'Enghien-les-Bains, l'eau des Fumades, ces eaux présentent notamment une concentration totale en carbonates ou bicarbonates supérieure à 360mg/l, sans que leur utilisation selon l'invention ne présente les inconvénients décrits ci-dessus.

On constate également que l'emploi de dioxyde de carbone dans le mélange de gaz propulseurs a une influence sur le pH de l'eau minérale ou thermale : en effet, lorsque, selon l'état de l'art, on utilise de l'azote comme gaz propulseur, on constate que le pH en sortie du dispositif est compris entre 7 et 8. La présence de CO₂ dissous dans l'eau acidifie le milieu, ce qui rapproche le pH de l'eau de celui de la peau (habituellement entre 5 et 6) et permet une meilleure tolérance du traitement, en particulier pour les peaux très sensibles.

En outre, des essais de différents types de diffuseurs ont permis d'optimiser la qualité du brumisat obtenu à l'aide d'un dispositif selon l'invention. De façon préférentielle, on utilise un diffuseur avec une buse munie de trois canaux tourbillonnaires. Le choix de ce diffuseur permet de mettre en oeuvre le dispositif selon l'invention avec un pourcentage élevé de dioxyde de carbone dans le mélange de gaz propulseur (jusqu'à 70% de CO₂) sans observer de jet en fin de restitution.

La valve est choisie pour assurer une alimentation suffisante du niveau du diffuseur sans risque de bouchage. De façon préférentielle, on choisit une valve utilisable tête en haut et tête en bas afin d'éviter les pertes de gaz en cas de mauvaise utilisation du bidon. Toutefois, une valve à utilisation exclusive tête en haut peut être envisagée.

Enfin, si le dioxyde de carbone est connu pour ses propriétés bactériostatiques, comme décrit notamment dans : 〈〈The inhibition by CO2 of the growth and metabolism of micro-organisms〉〉 N.M.Dixon, journal of applied bacteriology, 1989, 67, 109-136, il était inattendu que le mélange gazeux d'azote et de dioxyde de carbone, mis en oeuvre dans le dispositif selon l'invention, préférentiellement dans des proportions satisfaisant (Rel I), et de façon encore plus préférentielle (Rel II), permette d'observer une réduction du nombre de germes présents dans le milieu. L'utilisation d'un tel dispositif permet en outre, à partir d'une eau minérale ou d'une eau thermale, sans avoir recours à des méthodes physiques de décontamination, d'obtenir un milieu avec un niveau de contamination inférieur ou égal à 10 germes pour 100ml et de préférence inférieur ou égal à 1 germe pour 100ml en quelques jours (méthodes de comptage des germes données dans la Pharmacopée Européenne, 2^{e} édition, 1983, vol I, V.2.1.8).

Afin de mieux faire comprendre l'objet de la présente invention, on va décrire ci-après, à titre d'exemple, un dispositif répondant aux caractéristiques de cette invention.

Les figures 1a et 1b sont des vues en coupe longitudinale d'un dispositif de brumisation selon l'invention.

La figure 2 est une vue en coupe longitudinale du diffuseur à canaux tourbillonaires.

La figure 3 est une vue en coupe transversale du diffuseur à canaux tourbillonaires.

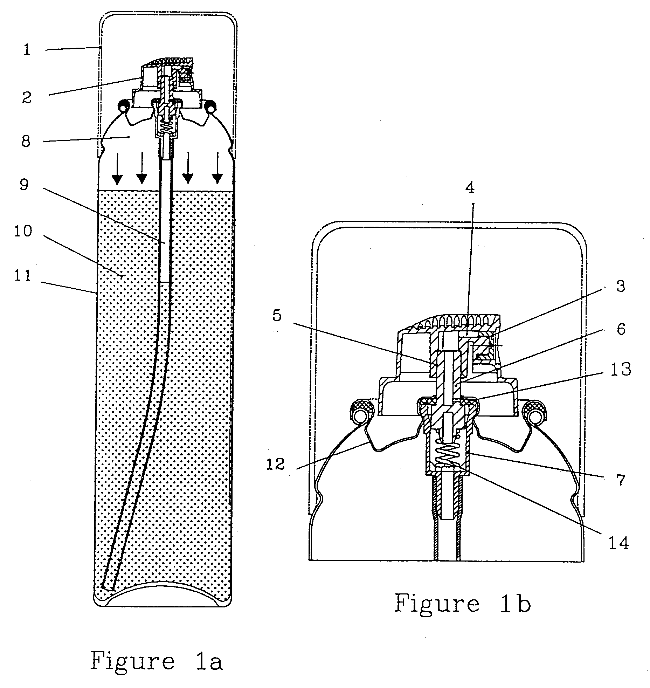

Un dispositif de brumisation individuel, représenté par les figures 1a et 1b, est constitué d'un récipient (11), contenant de l'eau (10) et un gaz propulseur (8), d'une valve et d'un moyen de distribution de l'eau relié à la valve ; la valve est constituée d'un corps de valve (7), d' une coupelle (12), d'une tige (6) d'actionnement de la valve et d'éjection de l'eau, d'un joint (13) et d'un ressort (14) ; le récipient (11) est surmonté d'un capot amovible (1) ; le moyen de distribution de l'eau est constitué d'un tube plongeur (9) fixé sur le corps de valve (7) et d'un bouton-poussoir (2), ce bouton-poussoir comprenant un emmanchement (5) qui s'emboite sur la tige (6), un canal de distribution (4) et une buse (3).

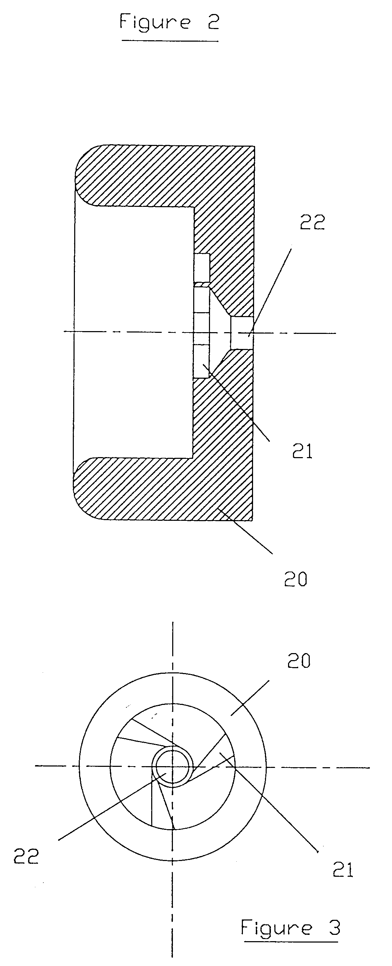

Sur les figures 2 et 3 on peut voir la buse (20) se terminant par un orifice (22) et comprenant trois canaux tourbillonaires (21).

### Essais

Sauf mention contraire, les essais sont réalisés avec un dispositif muni d'un diffuseur à trois canaux tourbillonnaires, de profondeur 0,20mm et possédant une buse plate, l'eau employée est une eau du bassin de Vichy (eau de Lucas).

Tous les pourcentages sont donnés en volume.
La pression initiale dans le dispositif est de 6.10⁵Pas, assurant un débit de 0,9g/s pour le diffuseur utilisé. En fin de restitution, la pression est de 2.10⁵Pas, le débit est alors de 0,5g/s.

### Essai 1 :

On a fait varier la proportion d'azote et de dioxyde de carbone et on a observé la formation ou l'absence de formation d'un jet en fin de restitution.
Le résultat est noté de 0 à 5 :
- 0: = pas de jet
- 1-2: = gouttes
- 3-4: = jet de faible puissance
- 5: = jet nettement formé

| | | | | | | | |
|---|---|---|---|---|---|---|---|
| Mélange % N₂ | 100 | 80 | 60 | 50 | 40 | 20 | 0 |
| gazeux %CO₂ | 0 | 20 | 40 | 50 | 60 | 80 | 100 |
| observations | 0 | 0 | 0 | 0 | 0 | 3 | 5 |

D'après ces essais, on a constaté qu'un gaz propulseur comprenant 70% ou moins de dioxyde de carbone ne provoquait pas la formation de jet.

### Essai 2:

On a évalué l'influence du dioxyde de carbone sur le pH en prélevant 5cm³ d'eau en sortie du dispositif à l'aide d'un diffuseur permettant une restitution rapide. La mesure est réalisée sur trois échantillons dont on a fait la moyenne.

| | | | | | | | |
|---|---|---|---|---|---|---|---|
| Mélange % N₂ | 100 | 80 | 60 | 50 | 40 | 20 | 0 |
| gazeux %CO₂ | 0 | 20 | 40 | 50 | 60 | 80 | 100 |
| pH | 7.6 | 6.9 | 6.7 | 6.6 | 6.6 | 6.6 | 6.5 |

Ces essais montrent que le dispositif selon l'invention permettait d'obtenir un brumisat dont le pH est plus proche de celui de la peau que les brumisats selon l'art antérieur.

### Essai 3:

On a évalué la formation d'un précipité de carbonate de calcium en mesurant par complexométrie (unité de mesure mg/l) le carbonate de calcium dissous dans l'eau en sortie de buse, en début (ligne A) et en fin de restitution (ligne B).

| | | | | | |
|---|---|---|---|---|---|
| Mélange % N₂ | 100 | 80 | 60 | 40 | 0 |
| gazeux %CO₂ | 0 | 20 | 40 | 60 | 100 |
| A | 53 | 165 | 176 | 170 | 165 |
| B | - | 31 | 161 | 163 | - |

Ces essais ont montré qu'un mélange gazeux d'azote et de dioxyde de carbone satisfaisant (Rel I) permettait d'éviter la précipitation de carbonates et de bicarbonates.

### Essai 4:

On évalue l'action du mélange gazeux sur la contamination bactérienne. Pour cela on a utilisé une eau physiologique stérilisée, dont la composition est sans influence sur la population bactérienne. On a introduit dans cette eau une quantité donnée de Pseudomonas aeruginosa ATCC19429. Cette contamination a été faite à partir de suspensions calibrées pour avoir des concentrations initiales de 10⁵ germes pour 100ml (C₁₀⁵) et de 10⁴ germes pour 100ml (C₁₀⁴). Cette eau a été conditionnée dans des dispositifs de brumisation selon l'invention dont le mélange gazeux est constitué de 50% d'azote et de 50% de dioxyde de carbone (D1). A titre comparatif, on a conditionné cette eau dans des dispositifs aérosols pressurisés par de l'azote (D2) et dans des récipients aérosols non pressurisés (D3). On a effectué un dénombrement par filtration des bactéries au moment de leur introduction dans l'eau (T₀), après 24 heures de stockage (T₂₄ₕ) et après sept jours de stockage (T₇ⱼ) à température ambiante (20±2°C). Le résultat est donné en nombre de germes par 100ml.

| | T₀ | T₂₄ₕ | T₇ⱼ |
|---|---|---|---|
| D1 C₁₀⁴ | 1,7.10⁴ | 3.10² | <5.10⁻¹ |
| D1 C₁₀⁵ | 8,5.10⁴ | 1,8.10² | <5.10⁻¹ |
| D2 C₁₀⁴ | 2,2.10⁴ | 1,3.10⁴ | 2,2.10² |
| D2 C₁₀⁵ | 1,5.10⁵ | 1,3.10⁵ | 1.10⁴ |
| D3 C₁₀⁴ | 1,7.10⁴ | 1,4.10⁴ | 3,5.10³ |
| D3 C₁₀⁵ | 8,5.10⁴ | 2,4.10⁵ | 4,3.10⁴ |

On constate que le dispositif selon l'invention permet de réduire le nombre de germes d'au moins 4 log, tandis que le dispositif pressurisé par de l'azote ne permet d'obtenir une réduction du nombre de germes que d'un seul log, tout comme le dispositif témoin (D3).

Des essais comparables ont permi de montrer que, dans des dispositifs selon l'invention, à température ambiante (20±2°C), la population microbienne d'une eau thermale restait stable pendant au moins neuf mois.

## Revendications

1. Dispositif de brumisation d'eau minéralisée constitué d'un récipient contenant l'eau minéralisée et un gaz propulseur, d'une valve et d'un moyen de distribution de l'eau, caractérisé en ce que le gaz propulseur est un mélange essentiellement constitué d'azote et de dioxyde de carbone.

2. Dispositif selon la revendication 1, caractérisé en ce que l'eau a un taux de contamination inférieur à 10 germes pour 100ml.

3. Dispositif selon la revendication 2, caractérisé en ce que l'eau a un taux de contamination inférieur ou égal à 1 germe pour 100ml.

4. Dispositif selon l'une quelconque des revendications 1 à 3, caractérisé en ce que le gaz propulseur est constitué d'au moins 30% d'azote.

5. Dispositif selon l'une quelconque des revendications 1 à 5, caractérisé en ce que le gaz propulseur est constitué d'au moins 20% de dioxyde de carbone.

6. Dispositif selon l'une quelconque des revendications 1 à 5, caractérisé en ce que le gaz propulseur est constitué d'au moins 40% de dioxyde de carbone.

7. Dispositif selon l'une quelconque des revendications 1 à 6, caractérisé en ce que les pourcentages d'azote et de dioxyde de carbone, en volume par rapport au volume gazeux total, satisfont la relation:
40/60 < %N₂/%CO₂ < 60/40 et %N₂ + %CO₂= 100 (Rel I)

8. Dispositif selon l'une quelconque des revendications 1 à 7, caractérisé en ce que les pourcentages d'azote et de dioxyde de carbone, en volume par rapport au volume gazeux total, satisfont la relation:
%N₂ = %CO₂= 50 (Rel II)

9. Dispositif selon l'une quelconque des revendications 1 à 8, caractérisé en ce que l'eau est une eau minérale ou thermale naturelle.

10. Dispositif selon l'une quelconque des revendications 1 à 9, caractérisé en ce que l'eau minéralisée est choisie parmi l'eau de Vittel, les eaux du bassin de Vichy, l'eau d'Uriage, l'eau de la Roche Posay, l'eau de la Bourboule, l'eau d'Enghien-les-Bains, l'eau de Saint Gervais-les-Bains, l'eau de Néris-les-Bains, l'eau d'Allevar-les-Bains, l'eau de Digne, l'eau de Maizières, l'eau de Neyrac-les-Bains, l'eau de Lons-le-Saunier, les Eaux Bonnes, l'eau de Rochefort, l'eau de Saint Christau, l'eau des Fumades et l'eau de Tercis-les-bains.

11. Dispositif selon l'une quelconque des revendications 1 à 10, caractérisée en ce que l'eau minéralisée contient au moins un adjuvant choisi parmi les conservateurs, les antioxydants, les parfums, les filtres, les matières colorantes, les actifs hydrophiles ou lipophiles.

12. Dispositif selon la revendication précédente, caractérisé en ce que l'eau minéralisée comprend de 0,01 % à 10 % en poids par rapport au poids total de la composition d'au moins un actif hydrophile ou lipophile.

13. Dispositif selon l'une quelconque des revendications 1 à 12, caractérisé en ce que l'eau minéralisée est une eau qui présente une concentration totale en carbonates ou en bicarbonates supérieure à 360mg/l.

14. Dispositif selon l'une quelconque des revendications 1 à 13, caractérisé en ce que le diffuseur est muni d'une buse comprenant trois canaux tourbillonaires.

15. Utilisation d'un mélange constitué essentiellement de dioxyde de carbone et d'azote comme gaz propulseur dans un dispositif de brumisation d'eau minérale.

16. Utilisation selon la revendication 15, caractérisée en ce que l'eau minérale a un taux de contamination a une valeur inférieure ou égale à 10 germes pour 100ml, préférentiellement 1 germe pour 100ml.

17. Utilisation selon la revendication 16, caractérisée en ce que l'eau est une eau minérale ou thermale naturelle.

18. Utilisation selon la revendication 17, caractérisée en ce que l'eau minérale ou thermale est choisie parmi l'eau de Vittel, les eaux du bassin de Vichy, l'eau d'Uriage, l'eau de la Roche Posay, l'eau de la Bourboule, l'eau d'Enghien-les-Bains, l'eau de Saint Gervais-les-Bains, l'eau de Néris-les-Bains, l'eau d'Allevar-les-Bains, l'eau de Digne, l'eau de Maizières, l'eau de Neyrac-les-Bains, l'eau de Lons-le-Saunier, les Eaux Bonnes, l'eau de Rochefort, l'eau de Saint Christau, l'eau des Fumades et l'eau de Tercis-les-bains.

## Claims

1. Spray device for mineralized water composed of a container containing mineralized water and a propellent gas, of a valve and of a means for dispensing the water, characterized in that the propellent gas is a mixture composed essentially of nitrogen and of carbon dioxide.

2. Device according to Claim 1, characterized in that the water has a degree of contamination of less than 10 germs per 100 ml.

3. Device according to Claim 2, characterized in that the water has a degree of contamination of less than or equal to 1 germ per 100 ml.

4. Device according to any one of Claims 1 to 3, characterized in that the propellent gas is composed of at least 30% of nitrogen.

5. Device according to any one of Claims 1 to 4, characterized in that the propellent gas is composed of at least 20% of carbon dioxide.

6. Device according to any one of Claims 1 to 5, characterized in that the propellent gas is composed of at least 40% of carbon dioxide.

7. Device according to any one of Claims 1 to 6, characterized in that the percentage of nitrogen and of carbon dioxide, by volume with respect to the total gas volume, satisfy the relationship:
40/60 < %N₂/%CO₂ < 60/40 and %N₂ + %CO₂ = 100 (Rel. I)

8. Device according to any one of Claims 1 to 7, characterized in that the percentages of nitrogen and of carbon dioxide, by volume with respect to the total gas volume, satisfy the relationship
%N₂ = %CO₂ = 50 (Rel. II)

9. Device according to any one of Claims 1 to 8, characterized in that the water is a natural mineral or thermal water.

10. Device according to any one of Claims 1 to 9, characterized in that the mineralized water is chosen from water from Vittel, waters from the Vichy basin, water from Uriage, water from La Roche Posay, water from La Bourboule, water from Enghien-les-Bains, water from Saint Gervais-les-Bains, water from Néris-les-Bains, water from Allevar-les-Bains, water from Digne, water from Maizières, water from Neyrac-les-Bains, water from Lons-le-Saunier, water from Eaux-Bonnes, water from Rochefort, water from Saint Christau, water from Les Fumades and water from Tercis-les-Bains.

11. Device according to any one of Claims 1 to 10, characterized in that the mineralized water contains at least one adjuvant chosen from preservatives, anti-oxidants, fragrances, screening agents, colouring materials, or hydrophilic or lipophilic active principles.

12. Device according to the preceding claim, characterized in that the mineralized water comprises from 0.01% to 10% by weight with respect to the total weight of the composition of at least one hydrophilic or lipophilic active principle.

13. Device according to any one of Claims 1 to 12, characterized in that the mineralized water is a water which exhibits a total concentration of carbonates or of bicarbonates greater than 360 mg/l.

14. Device according to any one of Claims 1 to 13, characterized in that the atomizer passage is equipped with a nozzle comprising three vortical channels.

15. Use of a mixture composed essentially of carbon dioxide and of nitrogen as propellent gas in a spray device for mineral water.

16. Use according to Claim 15, characterized in that the mineral water has a degree of contamination which has a value of less than or equal to 10 germs per 100 ml, preferably 1 germ per 100 ml.

17. Use according to Claim 16, characterized in that the water is a natural mineral or thermal water.

18. Use according to Claim 17, characterized in that the mineral or thermal water is chosen from water from Vittel, waters from the Vichy basin, water from Uriage, water from La Roche Posay, water from La Bourboule, water from Enghien-les-Bains, water from Saint Gervais-les-Bains, water from Néris-les-Bains, water from Allevar-les-Bains, water from Digne, water from Maizières, water from Neyrac-les-Bains, water from Lons-le-Saunier, water from Eaux-Bonnes, water from Rochefort, water from Saint Christau, water from Les Fumades and water from Tercis-les-Bains.

## Patentansprüche

1. Vorrichtung zum Zerstäuben von Mineralstoffe enthaltendem Wasser bestehend aus einem Behälter, der das Mineralstoffe enthaltende Wasser und ein Treibgas anthält, einem Ventil und einem Mittel zur Verteilung des Wassers,
**dadurch gekennzeichnet, daß**
das Treibgas ein Gemisch ist, das im wesentlichen aus Stickstoff und Kohlendioxid besteht.

2. Vorrichtung nach Anspruch 1, dadurch gekennzeichnet, daß das Wasser einen Kontaminationsgrad von unter 10 Keime pro 100 ml aufweist.

3. Vorrichtung nach Anspruch 2, dadurch gekennzeichnet, daß das Wasser einen Kontaminationsgrad von höchstons 1 Keim pro 100 ml aufweist.

4. Vorrichtung nach einem der Ansprüche 1 bis 3, dadurch gekennzeichnet, daß das Treibgas aus mindestens 30 % Stickstoff besteht.

5. Vorrichtung nach einem der Ansprüche 1 bis 4, dadurch gekennzeichnet, daß das Treibgas aus mindestens 20 % Kohlendioxid besteht.

6. Vorrichtung nach einem der Ansprüche 1 bis 5, dadurch gekennzeichnet, daß das Treibgas aus mindestens 40 % Kohlendioxid besteht.

7. Vorrichtung nach einem der Ansprüche 1 bis 6, dadurch gekennzeichnet, daß die Prozentanteile von Stickstoff und Kohlendioxid, ausgedrückt in Volumenprozent, bezogen auf das gesamte Gasvolumen, folgende Gleichung erfüllen;
40/60 < %N₂/%CO₂ < 60/40 und %N₂ + %CO₂ = 100 . (Gleichung I)

8. Vorrichtung nach einem der Ansprüche 1 bis 7, dadurch gekennzeichnet, daß die Prozentanteile von Stickstoff und Kohlendioxid, ausgedrückt in Volumenprozent, bezogen auf das gesamte Gasvolumen, folgende Gleichung erfüllen:
%N₂ = %CO₂ = 50 (Gleichung II).

9. Vorrichtung nach einem der Ansprüche 1 bis 8, dadurch gekennzeichnet, daß das Wasser ein natürliches Mineral- oder Thermalwasser ist.

10. Vorrichtung nach einem der Ansprüche 1 bis 9, dadurch gekennzeichnet, daß das Mineralstoffe enthaltende Wasser unter folgenden Wässern ausgewählt ist: Wasser aus Vittel, Wasser aus dem Vichy-Becken, Wasser aus Uriage, Wasser aus La Roche Posay, Wasser aus La Bourboule, Wasser aus Enghien-les-Bains, Wasser aus Saint Gervais-les-Bains, Wasser aus Néris-les-Bains, Wasser aus Allevar-les-Bains, Wasser aus Digne, Wasser aus Maizières, Wasser aus Neyrac-les-Beins, Wasser aus Lons-le-Saunier, Wasser aus Eaux Bonnes, Wasser aus Rochefort, Wasser aus Saint Cristau, Wasser aus Fumades und Wasser aus Tercis-les-Bains.

11. Vorrichtung nach einem der Ansprüche 1 bis 10, dadurch gekennzeichnet, daß das Mineralstoffe enthaltende Wasser mindestens einen Zusatzstoff enthält, der unter den Konservierungsmitteln, Antioxidantien, Parfums Filtern, Färbemitteln, den hydrophilen oder lipophilen Wirkstoffen ausgewählt ist.

12. Vorrichtung nach dem vorhergehenden Anspruch, dadurch gekennzeichnet, daß das Mineralstoffe enthaltende Wasser einen hydrophilen oder lipophilen Wirkstoff in einem Anteil von 0,01 bis 10 Gew.-%, bezogen auf das Gesamtgewicht der Zusammensetzung, enthält.

13. Vorrichtung nach einem der Ansprüche 1 bis 12, dadurch gekennzeichnet, daß das Mineralstoffe enthaltende Wasser ein Wasser ist, das eine Gesamtkonzentration an Carbonaten oder Hydrogencarbonaten über 360 mg/l aufweist.

14. Vorrichtung nach einem der Ansprüche 1 bis 13, dadurch gekennzeichnet, daß der Zerstäuber mit einer Spritzdüse ausgerüstet ist, die drei Verwirbelungskanäle besitzt.

15. Verwendung eines Gemisches, das im wesentlichen aus Kohlendioxid und Stickstoff besteht, als Treibgas in einer Zerstäubungsvorrichtung für Mineralwasser.

16. Verwendung nach Anspruch 15, dadurch gekennzeichnet, daß das Mineralwasser einen Kontaminationsgrad von höchstens 10 Keime pro 100 ml und vorzugsweise 1 Keim pro 100 ml aufweist.

17. Verwendung nach Anspruch 16, dadurch gekennzeichnet, daß das Wasser ein natürliches Mineral- oder Thermalwasser ist.

18. Verwendung nach Anspruch 17, dadurch gekennzeichnet, daß das Mineralwasser oder Thermalwasser unter folgenden Wässern ausgewählt wird: Wasser aus Vittel, Wasser aus dem Vichy-Becken, Wasser aus Uriage, Wasser auf La Roche Posay, Wasser aus La Bourboule, Wasser aus Enghien-les-Bains, Wasser aus Saint Gervais-les-Bains, Wasser aus Néris-les-Bains, Wasser aus Allevar-les-Bains, Wasser aus Digne, Wasser aus Maizières, Wasser aus Neyrac-les-Beins, Wasser aus Lons-le-Saunier, Wasser aus Eaux Bonnes, Wasser aus Rochefort, Wasser aus Saint Cristau, Wasser aus Fumades und Wasser aus Tercis-les-Bains.
